# FASCICULE DE BREVET EUROPEEN

(11) **EP 1 419 764 B1**
(45) Date de publication et mention de la délivrance du brevet: **01.08.2007**
(21) Numéro de dépôt: 03292767.5
(22) Date de dépôt: 05.11.2003
(51) Int. Cl.: A61K 8/64

(54) **Utilisation d'une association de composants à effet synergique inhibiteur de canaux calciques pour prévenir ou traiter les rides et les ridules**
Verwendung eines Assoziates aus synergistischen Kalziumkanalblocker-Agentien zur Vermeidung oder Behandlung von Falten und feinen Linien
Use of an association of synergic calcium channel blocker agents for preventing or treating wrinkles and fine lines

(30) Priorité: 13.11.2002 FR 0214183
(43) Date de publication de la demande: 19.05.2004
(73) Titulaire: L'ORÉAL, 75008 Paris (FR)
(72) Inventeur: Renault, Béatrice, 94410 Saint Maurice (FR)
(74) Mandataire: Allab, Myriam

(56) Documents cités:
- EP-A- 1 088 548
- EP-A- 1 180 524
- EP-A- 1 269 990
- WO-A-94/26308
- FR-A- 2 793 681

## Description

L'invention concerne une composition, adaptée à une application topique sur la peau, comprenant, dans un milieu physiologiquement acceptable, (i) au moins un peptide ou un mélange de peptides tels que définis dans la présente revendication 1 et (ii) au moins un inhibiteur de canaux calciques.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse, ou en prévenant la dégradation, des fibres élastiques qui composent le tissu cutané.
Bien que ces traitements permettent d'agir sur les rides et ridules dues au vieillissement chronologique ou intrinsèque, ainsi que sur celles dues au photo-vieillissement, ils n'ont pas d'effet sur les rides d'expression, lesquelles nécessitent une intervention sur la composante contractile des rides présente dans la peau.
Les rides d'expression sont en effet la résultante de mécanismes différents de ceux générant les rides dues au vieillissement.
Précisément, elles sont produites sous l'effet de la contrainte exercée sur la peau par les muscles peauciers qui permettent les mimiques. Selon la forme du visage, la fréquence des mimiques et les tics éventuels, elles peuvent apparaître dès l'enfance. L'âge, de même que certains facteurs environnementaux tels que l'exposition au soleil, n'intervient pas dans leur genèse mais peut les creuser davantage et les rendre permanentes.
Les rides d'expression se caractérisent par la présence de sillons sur le pourtour des orifices que constituent le nez (sillons nasogéniens), la bouche (rides para-buccales et rides dites de l'amertume) et les yeux (rides de la patte d'oie), autour desquels se situent les muscles peauciers, ainsi qu'entre les sourcils (rides de la glabelle ou du lion) et sur le front.

La demanderesse a montré que les muscles peauciers du visage, en particulier des fibres musculaires striées, qui se trouvent sous le contrôle direct de l'influx neuro-musculaire, jouaient un rôle essentiel dans la formation des rides d'expression et que la modulation de l'influx neuro-musculaire atténuait les rides d'expression et avait aussi un effet de "lissage" sur le microrelief cutané.
De plus, on pense que le phénotype de certains fibroblastes situés le long des lignes de tension créées sous l'effet des contractions des muscles peauciers lors des mimiques serait progressivement modifié sous l'effet de ces contractions, conférant ainsi à ces fibroblastes des propriétés contractiles particulières, assimilées aux propriétés du muscle strié.
Il est connu que les muscles peauciers du visage sont sous contrôle d'afférences nerveuses motrices du nerf facial et que, par ailleurs, les cloisons interlobulaires de l'hypoderme contiennent en leur sein des fibres qui constituent un tissu musculaire strié. Dans le système nerveux périphérique, la jonction entre un nerf et un muscle strié constitue la plaque neuro-musculaire, en amont de laquelle se trouve la voie nerveuse afférente appelée motoneurone. Par ailleurs, les membranes cellulaires de chaque fibre nerveuse mais également des cellules musculaires comportent de nombreux canaux ioniques, et notamment des canaux calciques, ou des canaux chlore, aptes à laisser traverser de façon contrôlée respectivement le Ca²⁺ ou le Cl⁻.

Les variations de la concentration de Ca²⁺ intracellulaire interviennent dans l'initiation des phénomènes électriques et mécaniques, par exemple dépolarisation, contraction des muscles lisses ou striés, sécrétion hormonale et activation d'enzymes.
En particulier, l'augmentation de la concentration de calcium est à l'origine de la contraction musculaire et sa diminution de la relaxation.
Il est généralement admis que pendant la phase de contraction, les filaments minces d'actine glissent entre les filaments épais de myosine entraînant ainsi le raccourcissement des sarcomères et par voie de conséquence un mouvement contractile de la cellule et globalement de la fibre.

Pour ce qui concerne les muscles striés squelettiques, à l'état relâché, l'actine n'est pas accessible aux ponts de myosine parce qu'elle est associée à un autre complexe protéique constitué par la troponine et la myosine.

Quand le calcium se lie au complexe troponine-myosine, les molécules d'actine deviennent accessibles et le phénomène contractile peut alors débuter. La molécule de troponine subit une modification de conformation qui révèle l'activité ATPase des têtes de la molécule de myosine dans les filaments épais initiant ainsi la contraction. L'hydrolyse de l'ATP en ADP et en phosphate fournit l'énergie chimique permettant aux filaments de glisser.
Le rôle du Ca²⁺ au niveau des protéines contractiles des muscles striés est donc un rôle activateur (désinhibiteur) de l'activité ATPasique.
Le relâchement du muscle strié a lieu quand les tubules transverses et la membrane cellulaire sont repolarisés, ceci permet le retour de la concentration intra-cytoplasmique cellulaire du Ca²⁺ à une valeur de 10⁻⁷ M, inférieure au seuil d'activation des enzymes intracellulaires telle que l'ATPase (seuil d'activation qui avoisine 1 à 2 logarithmes de concentration supérieure).

Dans la contraction des fibres des muscles lisses, le calcium n'est pas un activateur *per se* : il se combine à la calmoduline et le complexe calcium-calmoduline active la MLCK (myosin light chain kinase) en formant avec elle un complexe ternaire. Ce complexe transforme la myosine en myosine phosphorylée qui se combine à l'actine, entraînant une contraction des fibres lisses.

Le cycle contraction-relâchement est dû aux variations de la concentration du calcium cytoplasmique de 10⁻⁷ (inactif) à 10⁻⁵ M (actif).
La régulation de la concentration cytoplasmique intracellulaire du Ca²⁺ n'est possible que parce que les efflux cytoplasmiques de calcium corrigent les influx cytoplasmiques. Les échanges de Ca²⁺ intra-cytoplasmiques se font, soit vis-à-vis des vésicules de réserve intracellulaire, soit vis-à-vis de l'extérieur de la cellule. Dans les deux cas, le Ca²⁺ n'est pas disponible dans le cytoplasme. Ces échanges ne peuvent être assurés que par une expulsion du calcium cytoplasmique intracellulaire par un ou des mécanismes dits actifs, aptes à surmonter le gradient de potentiel électrochimique évoqué plus haut.

Deux types de mécanismes peuvent intervenir : une pompe à calcium qui expulse activement les cations aux dépens de l'hydrolyse d'ATP et un mouvement de Ca²⁺ couplé à un mouvement de Na⁺. Dans la plupart des cellules, la pompe à calcium ATP-dépendante opère plus efficacement en présence de la calmoduline qui augmente son affinité.
Afin de mieux décrire les changements de perméabilité au calcium, il est actuellement habituel de considérer que cette perméabilité correspond (i) à l'ouverture de canaux calciques dépendants du potentiel membranaire ou canaux VOC (voltage-operated channel) qui s'ouvrent lors de la dépolarisation et laissent entrer le calcium dans la cellule ou (ii) à l'activation de récepteurs membranaires.
Trois types de canaux VOC sont principalement connus : un canal s'ouvrant à un potentiel bas, le canal T (Transient ou Transitoire) et deux types de canaux s'ouvrant à un potentiel élevé, les canaux L (Long) et N (présent dans les neurones).
D'autre part, il est vraisemblable que ces canaux calciques présentent des spécificités tissulaires.
En 1965, T. Godfraind a mis en évidence que la perméabilité de la membrane au calcium pourrait être inhibée par des agents pharmacologiques, ce qui constituerait le mécanisme commun sur lequel agiraient des antagonistes du Ca²⁺.
On comprend donc de ce qui précède que la contraction ou l'hypercontraction de certains muscles de la face, ou de certaines cellules contractiles du derme comme les myofibroblates, supposées être impliquées dans la genèse des rides d'expression, peut être induite par différents mécanismes faisant notamment intervenir des échanges ioniques Cl⁻, Ca²⁺ et du calcium intracellulaire et qu'en agissant notamment sur les canaux calciques, il est possible de relâcher ces muscles ou ces cellules contractiles et de lisser ainsi les rides d'expression.

Jusqu'à présent, le moyen le plus couramment utilisé pour agir sur les rides d'expression est la toxine botulique qui est notamment injectée dans les rides de la glabelle qui sont les rides inter-sourcillières (voir J.D. Carruters et al., J. Dermatol. Surg. Oncol., 1992, 18, pp. 17-21). Les dermatologues ont également recours à des implants dégradables à base de collagène, d'acide hyaluronique ou d'acide polylactique.
Ces méthodes présentent toutefois l'inconvénient de nécessiter une intervention médicale et ne donnent pas de résultats durables.

Il existe donc un besoin de disposer de composés efficaces utilisables dans une composition adaptée à une application topique sur la peau pour prévenir ou traiter, notamment lisser ou estomper les rides et ridules, en particulier les rides d'expression.

Or, la Demanderesse a découvert avec étonnement que :
1) certains peptides ont pour effet d'inhiber les canaux calciques de type L, et que cet effet est observé sur les trois types de canaux calciques de type L (site DHP, site Diltiazem et site Vérapamil).
2) ces peptides associés au magnésium ont des effets antagonistes synergiques vis-à-vis des canaux calciques de type L, site vérapamil.

Ces canaux calciques de type L ont été identifiés au niveau des fibroblastes humains (Baumgarten LB and coll (1992), J. Biol. Chem, 267, 10524-10530 et Chen CF and coll (1988), Science, 239, 1024-1026). Les sites DHP (Dihydropyridine), Diltiazem et Vérapamil correspondent aux sites des canaux calciques de type L, spécifiques des agents pharmacologiques correspondants connus comme inhibiteurs calciques. Et chaque agent pharmacologique inhibiteur calcique a, en fonction de son affinité pour certains canaux calciques et pour certains tissus, un effet prédominant auquel correspond une indication thérapeutique préférentielle.

On connaît, certes, de la demande EP 1 180 524 des peptides à effet anti-rides qui, par un mécanisme d'inhibition du SNARE COMPLEX (SNAP Receptor Complex), entraînent la diminution de libération d'un neuro-médiateur, l'acétylcholine au niveau des espaces synaptiques.
Le mécanisme d'action de ces peptides est similaire à celui des toxines botuliques : ils affectent la formation et/ou la stabilité du complexe de protéines de fusion (SNARE), qui est un noyau de protéines membranaires constitué de SNAP 25 (Synaptosomal Associated Protein de 25kDa), syntaxine et synaptobrevine (ou VAMP pour Vesicle-Associated Membrane Protein) dont le rôle est de médier l'exocytose neuronale, c'est-à-dire la libération des neurotransmetteurs (Ferrer Montier et al, 1997 The Journal of Biological Chemistry, 272, 2634-2638).

Mais aucune activité inhibiteur de canaux calciques n'est décrite ni suggérée pour ces peptides. En outre, il n'est pas suggéré de les associer à un inhibiteur de canaux calciques.

Par ailleurs, on connaît le rôle du calcium et de la régulation de sa concentration intracellulaire dans les phénomènes de contraction/relâchement musculaires. Et il a été précédemment proposé pour relâcher ou relaxer les tissus, et ainsi diminuer les rides et les ridules, d'agir sur les canaux calciques (FR-2 793 681).

Mais aucune association entre (i) au moins un peptide ou mélange de peptides tel que défini ci-après et (ii) au moins un inhibiteur de canaux calciques n'a été jusqu'ici proposée.

Or, la Demanderesse a démontré qu'une telle association permet de neutraliser la formation des rides d'expression du visage : elle neutralise les effets des micro-tensions sur la peau, en relaxant les fibroblastes contractiles du derme supposés être impliqués dans la genèse des rides d'expression et permet ainsi d'estomper les rides d'expression et d'éviter qu'elles ne se creusent tout en respectant les expressions du visage.

La présente invention a donc pour objet une composition, adaptée à une application topique sur la peau, comprenant, dans un milieu physiologiquement acceptable, (i) au moins un peptide ou mélange de peptides comprenant un peptide choisi parmi le groupe formé par les peptides définis par SEQ ID N°2 et SEQ ID N°3

A titre de peptide préféré dans le cadre de l'invention, on peut utiliser l'hexapeptide défini par la SEQ ID N°2 (acétyl hexapeptide-3).

On peut également utiliser un peptide choisi parmi :
(i) un peptide substantiellement homologue au peptide défini par SEQ ID N°2 ;
(ii) un sel cosmétiquement acceptable dudit peptide défini par SEQ ID N°2 ;
(iii) un peptide défini par SEQ ID N°2 ayant subi des modifications chimiques réversibles.

L'hexapeptide mentionné ci-dessus est disponible auprès de la société LIPOTEC sous le nom commercial Argireline^{®} : il contient un enchaînement de 6 acides aminés : glutamyl- glutamyl- methyonyl- glutaminyl- arginyl- arginyl, le premier (N-terminal) étant acétylé et le dernier (C-terminal), amidé.

Par peptide ou séquence d'acides aminés « substantiellement homologue », on entend une séquence d'acides aminés identique à au moins 60%, de préférence à au moins 80% et encore plus préférentiellement à au moins 95% de la séquence SEQ ID N°2.

Par « pourcentage d'identité » entre deux séquences d'acides aminés au sens de la présente invention, on entend désigner un pourcentage de résidus d'acides aminés identiques entre les deux séquences à comparer, obtenu après le meilleur alignement, ce pourcentage étant purement statistique et leurs différences entre les deux séquences étant réparties au hasard et sur toute leur longueur. On entend désigner par « meilleur alignement « ou « alignement optimal », l'alignement pour lequel le pourcentage d'identité déterminé comme ci-après est le plus élevé. Les comparaisons de séquences entre deux séquences d'acides aminés sont traditionnellement réalisées en comparant ces séquences après les avoir alignées de manière optimale, ladite comparaison étant réalisée par segment ou par « fenêtre de comparaison » pour identifier et comparer les régions locales de similarité de séquence. L'alignement optimal des séquences pour la comparaison peut être réalisé, outre manuellement, au moyen de l'algorithme d'homologie locale de Smith et Waterman (1981, Ad. App. Math. 2 :482), au moyen de l'algorithme d'homologie locale de Neddleman et Wunsch (1970, J.Mol. Biol. 48: 443), au moyen de la méthode de recherche de similarité de Pearson et Lipman (1988, Proc. Natl. Acad. Sci. USA 85 :2444), au moyen de logiciels informatiques utilisant ces algorithmes (GAP, BESTFIT, BLAST P, BLAST N disponible sur le site NCBI, FASTA et TFASTA dans le Wisconsin Genetics Software Package, Genetics Computer Group, 575 Science Dr, Madison, WI). Afin d'obtenir l'alignement optimal, on utilise de préférence le programme BLAST, avec la matrice BLOSUM 62. On peut également utiliser les matrices PAM ou PAM2590.
Le pourcentage d'identité entre deux séquences d'acides aminés est déterminé en comparant ces deux séquences alignées de manière optimale dans laquelle la séquence d'acides aminés à comparer peut comprendre des additions ou des délétions par rapport à la séquence de référence pour un alignement optimal entre ces deux séquences. Le pourcentage d'identité est calculé en déterminant le nombre de positions identiques pour lesquelles le résidu d'acide aminé est identique entre les deux séquences, en divisant ce nombre de positions identiques par le nombre de positions comparées et en multipliant le résultat obtenu par 100 pour obtenir le pourcentage d'identité entre ces deux séquences.

Par « sel cosmétiquement acceptable dudit peptide », on entend des sels métalliques ou des sels formés par l'addition d'acides ou de bases appropriées, pouvant être obtenus à partir d'une réaction avec les peptides selon l'invention selon les méthodes connues de l'homme du métier.
Comme sels organiques de peptide, on peut citer le gluconate de peptide ou l'acétate de peptide ou le citrate de peptide ou l'oléate de peptide ou l'oxalate de peptide.
Comme sels inorganiques de peptide, on peut citer les sels minéraux comme le chlorure de peptide ou le borate de peptide ou le sulfate de peptide ou le carbonate de peptide.

Comme « modifications chimiques réversibles » dudit peptide de sorte à augmenter sa biodisponibilité et sa facilité à passer à travers le tissu épithélial sans affecter sa capacité à inhiber les canaux calciques de type L, on peut citer par exemple la réaction d'estérification de groupes carboxylates des acides aminés glutamique et aspartique avec un groupe acétyl-méthyl, éliminant la charge négative de l'acide aminé et augmentant son hydrophobicité.

Ces peptides peuvent être obtenus par des méthodes conventionnelles de synthèse peptidique chimique ou des méthodes basées sur la technologie d'ADN recombinant, bien connues de l'homme du métier. On peut par exemple utiliser la méthode de synthèse chimique en phase solide décrite par Pennington et al. (1994, Peptide synthesis protocols, Humana Press, Totowa).

On peut également utiliser dans le cadre de l'invention tout peptide ou mélange de peptides choisi parmi :
a) un mélange de peptides comprenant au moins un peptide choisi parmi le groupe formé par les peptides définis par SEQ ID N°2 et SEQ ID N°3 et au moins un peptide de 3 à 30 acides aminés contenu dans SEQ ID N°4 (séquence du peptide (COOH)) ;
b) un mélange de peptidescomprenant au moins un peptide choisi parmi le groupe formé par les peptides définis par SEQ ID N° 2 et SEQ ID N°3 (domaine N-terminal) et d'au moins un peptide choisi parmi le groupe formé par les peptides définis par SEQ ID N°5 et SEQ ID N°6 (domaine C-terminal).

Ces peptides seront, selon l'invention, associés à un autre inhibiteur de canaux calciques, dont l'activité sera potentialisée. Comme inhibiteurs de canaux calciques, on connaît deux classes d'actifs, respectivement :
1) les agents actifs sur la membrane plasmique, inhibiteurs de l'entrée du calcium ;
2) les agents actifs à l'intérieur de la cellule (libération des réserves intracellulaires du Ca²⁺ ou alors inhibition de la formation du complexe Ca²⁺-Calmoduline.

Pour qu'une substance soit reconnue comme un inhibiteur des canaux calciques, autrement appelé dans le texte antagoniste calcique, elle doit pouvoir diminuer la concentration intracellulaire en calcium ou diminuer la liaison du calcium aux protéines intracellulaires comme par exemple la calmoduline, tel que cela est notamment décrit par exemple, par Galizzi, J.P et al, J. Biol. Chem. 1987, 262 p 6947 ou Y. Okamiya et al, Eur. J. Pharmacol. 1991, 205, p 49 ou J.A. Wagner et al, J. Neurosci. 1988, 8, p 3354 ou H.R Lee et al, Life Sci. 1984, 35 p 721 ou Schoemaker H. et Lauger S. Eur. J. Pharmacol. 1985, 111 p 273 ou encore I.J. Reynolds et al, J. Pharmacol. Exp. Ther. 1986, 237 p 731.

Une substance est reconnue comme relaxante lorsqu'elle montre un effet de relaxation sur un tissu musculaire contracté et/ou montre un effet inhibiteur dans un modèle expérimental de jonction nerf-muscle (plaque motrice) notamment dans le modèle décrit par W. Steinbrecher dans : Electrodes for stimulation and bioelectric potential recording, Ed. Biomerstechnich, 1988, pages 96-98.

Préférentiellement selon l'invention, on utilise des agents actifs sur la membrane plasmique, inhibiteurs de l'entrée du calcium ou encore complexant le calcium, comme l'alvérine et/ou ses sels organiques ou inorganiques, le manganèse et/ou ses sels organiques ou inorganiques ou encore le magnésium et/ou ses sels.

Ces composés peuvent être d'origine naturelle ou synthétique. Par "origine naturelle", on entend un composé à l'état pur ou en solution quelle que soit sa concentration dans ladite solution, qui peut être obtenu selon différents procédés d'extraction à partir d'un produit naturel. Par "origine synthétique", on entend un composé à l'état pur ou en solution à toute concentration, obtenu par synthèse chimique.

On peut donc utiliser l'alvérine et/ou ses sels organiques ou inorganiques. Comme sels organiques d'alvérine utilisables selon l'invention, on peut citer le gluconate d' alvérine ou l'acétate d' alvérine ou le citrate d' alvérine ou l'oléate d' alvérine ou l'oxalate d' alvérine.
Comme sels inorganiques d' alvérine, on peut citer les sels minéraux comme le chlorure d' alvérine ou le borate d' alvérine ou le nitrate d' alvérine ou le phosphate d' alvérine ou le sulfate d' alvérine ou le carbonate d' alvérine. Préférentiellement selon l'invention, le sel organique est le citrate d' alvérine et le sel inorganique est le chlorure d' alvérine.

On peut également utiliser le manganèse, qu'il soit sous la forme ionique ou sous la forme de sel ou sous la forme d'extraits naturels, végétaux ou de micro-organismes, particulièrement bactériens, riches en manganèse.
Comme sels organiques de manganèse, on peut citer le gluconate de manganèse ou le carbonate de manganèse ou l'acétate de manganèse ou le citrate de manganèse ou l'oléate de manganèse ou l'oxalate de manganèse.
Comme sels inorganiques de manganèse on peut citer les sels minéraux comme le chlorure de manganèse ou le borate de manganèse ou le nitrate de manganèse ou le phosphate de manganèse ou le sulfate de manganèse.
Bien entendu si l'on utilise un extrait naturel, végétal ou de micro-organismes, particulièrement bactériens, riche en manganèse, l'homme du métier sait adapter la quantité d'extrait à utiliser pour, au final, utiliser le manganèse dans les quantités appropriées pour l'effet recherché.
Comme extraits naturels riches en manganèse utilisables selon l'invention, on peut citer les extraits de noix ou les extraits de thé.

Comme inhibiteur calcique préféré, on utilisera dans le cadre de l'invention le magnésium ou ses sels.
Les effets du magnésium, déduits notamment d'observations de déficience et de surcharge, sont généralement antagonistes de ceux du calcium. Le magnésium est connu pour inhiber des canaux cationiques, sodium et surtout calcium récepteur et voltage-dépendants, et se comporte comme un anticalcique (M.L. Olinger, Disorders of calcium and magnesium metabolism, The Emergency Medecine Clinics of North America, Vol 7, N°4, November 1989).
Comme sels de magnésium préférés, on peut citer le sulfate de magnésium, le gluconate de magnésium, l'aspartate de magnésium, le chlorure de magnésium et le pidolate de magnésium.

La quantité des composés utilisables selon l'invention est bien entendu fonction de l'effet recherché et peut donc varier dans une large mesure.

Pour donner un ordre de grandeur, on peut utiliser le peptide ou le mélange de peptides en une quantité représentant de 0,000001% à 1% du poids total de la composition, préférentiellement en une quantité représentant de 0,00001% à 0,01% du poids total de la composition.

De même il est possible d'utiliser selon l'invention un inhibiteur de canaux calciques en une quantité représentant de 0,0001 % à 10% du poids total de la composition et préférentiellement en une quantité représentant de 0,01 % à 1 % du poids total de la composition.

La composition selon l'invention est adaptée à une application topique sur la peau et elle contient donc un milieu physiologiquement acceptable, c'est-à-dire compatible avec la peau et éventuellement avec ses phanères (cils, ongles, cheveux) et/ou les muqueuses.

Cette composition peut se présenter sous toutes les formes galéniques normalement utilisées dans le domaine cosmétique , et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées de l'association d'actifs anti-rides selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les hydrocarbures d'origine minérale ou synthétique (huile de vaseline, polyisobutène hydrogéné), les huiles d'origine végétale (huile d'avocat, huile de soja), les huiles d'origine animale (lanoline), les huiles siliconées (cyclométhicone, diméthicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides et en particulier l'acide polyacrylamido méthylpropane sulfonique réticulé, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.
Un agent hydrophile préféré pour la composition selon l'invention est choisi parmi un polyacide acrylamido methylpropane sulfonique tel que décrit dans EP 0850642 ou WO9800094 ou l'Hostacerin AMPS commercialisé par Clariant.

Comme actifs, il sera avantageux d'introduire dans la composition utilisée selon l'invention au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation et en particulier les agents stimulant la synthèse de macromolécules épidermiques tels qu'un extrait de bourgeons de hêtre (notamment celui commercialisé par la société GATTEFOSSE sous la dénomination commerciale Gatuline), les agents stimulant la synthèse de collagène tels que les hydrolysats de protéine de soja (notamment celui commercialisé par la société COLETICA sous la dénomination commerciale Phytokine), les agents stimulant la synthèse d'élastine et/ou inhibant la dégradation du collagène tels qu'un extrait d'algue *Macrocystis pyrifera* (notamment celui commercialisé par la société SECMA sous la dénomination commerciale Kelpadelie) et les agents stimulant la synthèse de glycosaminoglycanes tels qu'un extrait de *Saccharomyces cerevisiae* (notamment celui commercialisé par la société COGNIS sous la dénomination commerciale Cytovitin) ; les agents stimulant la prolifération des fibroblastes et/ou des kératinocytes ou stimulant la différenciation des kératinocytes et en particulier un extrait protéique de soja tel que celui commercialisé par la société COGNIS sous la dénomination commerciale Eleseryl ; les agents dermo-relaxants tels que les sapogénines ou les extraits naturels, tels qu'un extrait de Wild Yam ; les agents tenseurs tels que les polymères comportant un squelette polysiloxane sur lequel sont greffés des motifs polymères mixtes du type acide poly(méth)acrylique et du type poly(méth)acrylate d'alkyle et en particulier ceux commercialisés par la société 3M sous les dénominations commerciales LO21 et VS80 ; les agents anti-pollution et/ou anti-radicalaire ; les agents agissant sur la microcirculation ; les agents agissant sur le métabolisme énergétique des cellules ; et leurs mélanges.

Les compositions conformes à l'invention peuvent comporter en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Les agents photoprotecteurs organiques plus particulièrement préférés sont choisis parmi les composés suivants : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyle)-s-triazine, Anisotriazine, Ethylhexyl triazone, Diethylhexyl Butamido Triazone, Methylène bis-Benzotriazolyl Tetramethylbutylphénol, Drometrizole Trisiloxane, 1,1-dicarboxy (2,2'-diméthyl-propyl)-4,4-diphénylbutadiène, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe
ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

L'invention concerne également l'utilisation cosmétique d'au moins une association telle que décrite précédemment, dans une composition adaptée à une application topique sur la peau, en tant qu'agent pour lisser les rides et ridules, en particulier les rides d'expression.

L'invention a aussi pour objet un procédé de traitement cosmétique d'une peau ridée, comprenant l'application topique sur ladite peau d'une composition selon l'invention, en particulier sur les zones du visage ou du front marquées par des rides d'expression et/ou sur les personnes présentant des rides et d'expression.

Selon un procédé particulier de l'invention, la composition est appliquée sur les rides et ridules disposées radialement autour de la bouche et/ou des yeux et/ou horizontalement sur le front et/ou situées dans l'espace inter-sourcillier.

L'invention sera maintenant illustrée par les exemples non limitatifs suivants. Dans ces exemples, les quantités sont indiquées en pourcentage pondéral.

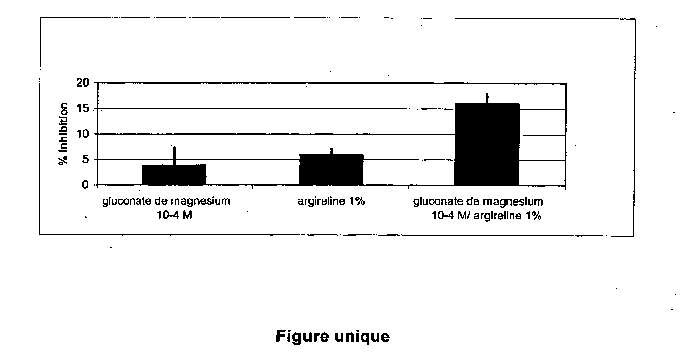

La figure est un histogramme représentant l'effet du gluconate de magnésium (10⁻⁴ M) et de l'Argireline^{®} 1% sur les canaux calciques ainsi que l'effet de leur association sur les canaux calciques de type L, site vérapamil.

### EXEMPLES

### Exemple 1 : Mise en évidence d'un effet inhibiteur de l'Argireline sur les canaux calciques de type L, et d'un effet synergique inhibiteur de la combinaison Argireline-Magnésium sur les canaux calciques de type L, site vérapamil

Le test mesure la capacité d'un produit à inhiber par compétition la fixation d'agonistes de canaux calciques de type L.
Les études sont réalisées à partir d'homogénats de cortex cérébral de rat (membranes isolées présentant à leur surface des canaux calciques de type L).

Le principe d'une expérience de déplacement à l'équilibre consiste à mesurer la liaison spécifique à l'équilibre, d'une concentration donnée en ligand radiomarqué en présence d'une concentration variable et croissante de ligand froid. Le ligand froid entre en compétition avec le ligand radioactif pour sa liaison au récepteur, c'est pourquoi on peut parler de compétition de la liaison à l'équilibre. Cette technique permet :
- de démontrer qu'un ligand froid se lie à un récepteur
- d'étudier la liaison d'un ligand de faible affinité pour un récepteur.

Les produits testés sont répertoriés dans le tableau 1 suivant :

**Tableau 1**

| Composé | PM | Solution stock |
|---|---|---|
| Argireline^{®} 1 % | | 100 % (v/v) dans l'eau |
| Sulfate ou Gluconate de Magnésium 10-4 M | 414.3 | 1. 10⁻² M dans l'eau |
| Mélange gluconate de magnésium 10⁻⁴ M et Argireline® 1 % | - | 1. 10⁻³ M dans l'eau |

Les conditions expérimentales selon le protocole décrit par Reynolds I.J. et al., 1986, *J. Pharmacol. Exp. Ther*., 237, p.731, sont présentées dans le tableau 2 ci-dessous :

**Tableau 2**

| Test | Ligand | Conc. | Non spécifique | Incubation | Détection |
|---|---|---|---|---|---|
| Canal Ca ²⁺ (L, site verapamil) | (³ H)(-) (³H)(-) D 888 | 0.5 nM 0.5 nM | D 600 (1 0 µM) D 600 (10 µM) | 60 min. / 22°C 60 min. / 22°C | Comptage scintillation |

D888 et D600 sont les molécules de référence spécifiques des canaux calciques de type L, site vérapamil.

La liaison spécifique d'un ligand (D888 marqué) aux récepteurs (canaux calciques de type L, site vérapamil) est défini comme la différence entre la liaison totale et la liaison spécifique déterminée en présence d'un excès de ligand froid. Les résultats sont exprimés en pourcentage de liaison spécifique et en pourcentage d'inhibition de liaison spécifique en présence des composés testés (Argireline^{®}).

Les résultats obtenus pour le site Vérapamil sont reportés dans le tableau 3 ci-dessous et la figure en annexe. Le pourcentage de liaison (%) et les pourcentages d'inhibition moyen sont indiqués

**Tableau 3**

| Composés | Concentration | % de liaison | | | % de liaison moyen | % inhibition moyen |
|---|---|---|---|---|---|---|
| Argireline® | 1.0 % | 95.1 | 93.1 | 99.9 | 96.1 | 4 |
| Gluconate de magnésium | 1.10-⁴ M | 93.8 | 95.2 | 93.0 | 94.0 | 6 |
| Gluconate de magnésium/ Argireline® | 1.10-⁴M/1.0 % | 81.6 | 85.3 | 85.1 | 84.0 | 16 |

Le % de liaison correspond au pourcentage de liaison du ligand en présence d'Argireline^{®}, qui joue le rôle de compétiteur au niveau du site Vérapamil. Ces résultats montrent donc que l' Argireline^{®} inhibe les canaux calciques de type L, site Vérapamil. L'effet est observé principalement aux deux concentrations les plus élevées, 5% et 10 % avec des pourcentages d'inhibition moyens de 36 % et 52 % respectivement. A la concentration de 1 %, Argireline présente un effet modéré (4%).

Le gluconate de Magnésium utilisé seul à des concentrations de 1.10⁻⁶ à 1.10⁻⁴ M a des effets modérés sur les canaux calciques de type L, site vérapamil. A la concentration la plus élevée, le pourcentage d'inhibition moyen est de 6%.

De façon surprenante, on note que l'association Argireline® 1% - Gluconate de Magnésium 10⁻⁴ M présente un effet synergique inhibiteur de canaux calciques de type L, site vérapamil. L'effet représente une inhibition moyenne de 16 %, soit un effet supérieur à la somme des effets de chacun des composés.

### Exemple 2 : Exemples de formulations

Les compositions suivantes sont préparées de manière classique pour l'homme du métier. Les quantités indiquées sont en pourcentages pondéraux.

### COMPOSITION N° 1 : EMULSION H/E

| | | |
|---|---|---|
| **Phase A** | Eau | Qsp 100% |
| | Conservateurs | 0,35 % |
| | Gluconate de manganèse | 0,05 % |
| | | |
| **Phase B1** | Huiles de silicone | 3 % |
| | Alcools cétylique et stéarylique | 1,6 % |
| | Polyisobutène hydrogéné | 4 % |
| | Polyéthylène glycol (100 OE) | 2 % |
| **Phase B2** | Huiles de silicone volatiles | 3 % |
| | | |
| **Phase D** | Acide polyacrylamido méthylpropane sulfonique réticulé (Hostacerin AMPS de CLARIANT) | 1 % |
| | | |
| **Phase E** | Hexapeptide acetyl glutamyl-glutamyl-methionyl-glutaminyl-arginyl-arginylamide à 0,05% dans l'eau (Argireline de LIPOTEC) | 1 % |

### COMPOSITION N° 2 : ÉMULSION E/H

| | | |
|---|---|---|
| **Phase A** | Eau | Qsp 100% |
| | Acide Citrique | 0,05 % |
| | Conservateurs | 0,95 % |
| | Hydroxyde de Sodium à 50 % | 2,98 % |
| | Copolymère vinylpyrrolidone/styrene en émulsion à 40% | 3,34 % |
| | Polysaccharide riche en fucose à 1 % dans l'eau | 2 % |
| | (Fucogel de SOLABIA) | |
| | Mono-laurate de sorbitane oxyéthyléné (20 OE) | 1 % |
| | Citrate de sodium | 1,6 % |
| | Gluconate de magnésium | 0,05 % |
| | Hexapeptide acetyl glutamyl-glutamyl-methionyl- | 1 % |
| | glutaminyl-arginyl-arginylamide à 0,05% dans l'eau (Argireline de LIPOTEC) | |
| | | |
| **Phase B** | Mélange de cyclopentasiloxane et polydimétrhylsiloxane oxyéthyléné (18OE) oxypropyléné (18 OP) | 10 % |
| | Cyclopentasiloxane | 8 % |
| | Elastomère de siloxane à 20% dans le polydiméthylsiloxane (KSG-1 de SHIN-ETSU) | 3% |
| **Phase C** | Copolymère acrylamide / acrylamido-2-méthyl propane sulfonate de sodium en émulsion inverse à 40% (Sepigel 305 de SEPPIC) | 2 % |

### COMPOSITION N° 3 : GEL

| | |
|---|---|
| Glycérine | 4 % |
| Propylène glycol | 3 % |
| Conservateurs | Qsp |
| Copolymère acrylamide / acrylamido-2-méthyl propane sulfonate de sodium en émulsion inverse à 40 % (Sepigel 305 de SEPPIC) | 1,5% |
| Poudre de copolymère méthacrylate | 1 % |
| Alvérine | 0,1 % |
| Hexapeptide acetyl glutamyl-glutamyl-methionyl-glutaminyl-arginyl-arginylamide à 0,05% dans l'eau (Argireline de LIPOTEC) | 4 % |
| Eau | Qsp 100 % |

### COMPOSITION N° 4 : SERUM

| | |
|---|---|
| Hydroxyde de sodium | 0,1 % |
| Acétate de tocophérol | 0,5 % |
| Sel disodique d'EDTA | 0,1 % |
| Polyisobutène hydrogéné | 4,0 % |
| Caféine | 0,2 % |
| Gluconate de magnésium | 0,5 % . |
| Glycérine | 3,0 % |
| Conservateurs | 1,2 % |
| Cyclohexadiméthylsiloxane | 6,0 % |
| Acide polyacrylamido méthylpropane sulfonique réticulé (Hostacerin AMPS de CLARIANT) | 1,0 % |
| Extrait de levure | 0,3% |
| Hexapeptide acetyl glutamyl-glutamyl-methionyl-glutaminyl-arginyl-arginylamide à 0,05% dans l'eau (Argireline de LIPOTEC) | 1,0 % |
| Acide citrique | 0,1 % |
| Agents tenseurs | 7,0 % |
| Eau qsp | 100 % |

Dans chacune des formulations précédentes, l'Argireline^{®} peut être remplacée par l'un des autres peptides utilisables selon l'invention.

Les compositions ci-dessus sont destinées à être appliquées sur le visage le matin et/ou le soir pour corriger les rides et ridules d'expression et détendre les traits.

## Revendications

1. Composition, adaptée à une application topique sur la peau, comprenant, dans un milieu physiologiquement acceptable, (i) au moins un peptide ou un mélange de peptides comprenant un peptide choisi parmi le groupe formé par les peptides définis par SEQ ID N° 2 et SEQ ID N°3, ou parmi
- un peptide substantiellement homologue au peptide défini par SEQ ID N°2 ;
- un sel cosmétiquement acceptable au peptide défini par SEQ ID N°2 ;
- un peptide défini par SEQ ID N°2 ayant subi des modifications chimiques réversibles
et (ii) au moins un inhibiteur de canaux calciques.

2. Composition selon la revendication 1, dans laquelle le peptide est l'hexapeptide défini par la SEQ ID N°2.

3. Composition selon la revendication 1, dans laquelle le mélange de peptides est choisi parmi :
a. un mélange de peptides comprenant au moins un peptide choisi parmi le groupe formé par les peptides définis par SEQ ID N° 2 et SEQ ID N°3 et au moins un peptide de 3 à 30 acides aminés contenu dans SEQ ID N°4 ;
b. un mélange de peptides comprenant au moins un peptide choisi parmi le groupe formé par les peptides définis par SEQ ID N°2 et SEQ ID N°3 et au moins un peptide choisi parmi le groupe formé par les peptides définis par SEQ ID N°5 et SEQ ID N°6.

4. Composition selon l'une quelconque des revendications 1 à 3, dans laquelle l'acide aminé de l'extrémité N-terminale dudit peptide tel que défini dans l'une des revendications 1 à 3 est acétylé et/ou l'acide aminé de son extrémité C-terminale est amidé.

5. Composition selon l'une quelconque des revendications 1 à 4, **caractérisée en ce que** l'inhibiteur de canaux calciques est choisi parmi l'alvérine et/ou ses sels, le manganèse et/ou ses sels, le magnésium et/ou ses sels.

6. Composition selon l'une quelconque des revendications 1 à 5, **caractérisé en ce que** l'inhibiteur de canaux calciques est un sel de magnésium choisi parmi le gluconate de magnésium, le sulfate de magnésium, l'aspartate de magnésium, le chlorure de magnésium et le pidolate de magnésium.

7. Composition selon l'une quelconque des revendications 1 à 6, **caractérisé en ce qu'**elle comprend au moins l'hexapeptide défini par la séquence SEQ ID N°2 dans laquelle l'acide aminé de l'extrémité N-terminale est acétylé et l'acide aminé de l'extrémité C-terminale est amidé et au moins le gluconate de magnésium.

8. Composition selon l'une quelconque des revendications 1 à 7, **caractérisée en ce que** ledit peptide ou le mélange de peptides représente de 0,000001% à 1% du poids total de la composition.

9. Composition selon l'une quelconque des revendications 1 à 8, **caractérisée en ce que** ledit peptide ou le mélange de peptides représente de 0,00001% à 0,01% du poids total de la composition.

10. Composition selon l'une quelconque des revendications 1 à 9, **caractérisée en ce que** l'inhibiteur de canaux calciques représente de 0,0001 % à 10 % du poids total de la composition.

11. Composition selon l'une quelconque des revendications 1 à 10, **caractérisée en ce que** l'inhibiteur de canaux calciques représente de 0,01 % à 1 % du poids total de la composition.

12. Composition selon l'une quelconque des revendications 1 à 11, **caractérisée en ce que** ladite composition renferme en outre au moins un actif choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents dermo-relaxants ; les agents tenseurs ; les agents anti-pollution et/ou anti-radicalaire ; les agents agissant sur la microcirculation ; les agents agissant sur le métabolisme énergétique des cellules ; et leurs mélanges.

13. Utilisation cosmétique (i) d'au moins un peptide ou mélange de peptides tels que définis dans l'une des revendications 1 à 4 et (ii) d'au moins un inhibiteur de canaux calciques tel que défini dans l'une des revendications 1, 5 ou 6, dans une composition adaptée à une application topique sur la peau, comme agent pour prévenir ou traiter, en particulier lisser, les rides et ridules.

14. Utilisation selon la revendication 13, **caractérisée en ce que** lesdites rides sont des rides d'expression.

15. Procédé de traitement cosmétique d'une peau ridée, comprenant l'application topique sur ladite peau d'une composition selon l'une quelconque des revendications 1 à 12.

16. Procédé selon la revendication 15, **caractérisé en ce que** ladite composition est appliquée sur les zones du visage ou du front marquées par des ridules et des rides d'expression et/ou sur les personnes présentant des ridules et des rides d'expression.

17. Procédé selon l'une des revendications 15 ou 16, **caractérisé en ce que** ladite composition est appliquée sur les ridules et rides disposées radialement autour de la bouche et/ou des yeux et/ou horizontalement sur le front et/ou situées dans l'espace inter-sourcillier.

## Claims

1. Composition, suitable for topical application to the skin, comprising, in a physiologically acceptable medium, (i) at least one peptide or mixture of peptides comprising a peptide chosen from the group formed by the peptides defined by SEQ ID No. 2 and SEQ ID No. 3, or from
- a peptide that is substantially homologous with the peptide defined by SEQ ID No. 2;
- a cosmetically acceptable salt of the peptide defined by SEQ ID No. 2;
- a peptide defined by SEQ ID No. 2 that has undergone reversible chemical changes; and (ii) at least one calcium-channel inhibitor.

2. Composition according to Claim 1, in which the peptide is the hexapeptide defined by SEQ ID No. 2.

3. Composition according to Claim 1, in which the peptide mixture is chosen from:
a. a peptide mixture comprising at least one peptide chosen from the group formed by the peptides defined by SEQ ID No. 2 and SEQ ID No. 3 and at least one peptide of 3 to 30 amino acids contained in SEQ ID No. 4;
b. a peptide mixture comprising at least one peptide chosen from the group formed by the peptides defined by SEQ ID No. 2 and SEQ ID No. 3 and at least one peptide chosen from the group formed by the peptides defined by SEQ ID No. 5 and SEQ ID No. 6.

4. Composition according to any one of Claims 1 to 3, in which the amino acid of the N-terminal end of the said peptide as defined in one of Claims 1 to 3 is acetylated and/or the amino acid of its C-terminal end is amidated.

5. Composition according to any one of Claims 1 to 4, **characterized in that** the calcium-channel inhibitor is chosen from alverine and/or its salts, manganese and/or its salts, and magnesium and/or its salts.

6. Composition according to any one of Claims 1 to 5, **characterized in that** the calcium-channel inhibitor is a magnesium salt chosen from magnesium gluconate, magnesium sulfate, magnesium aspartate, magnesium chloride and magnesium pidolate.

7. Composition according to any one of Claims 1 to 6, **characterized in that** it comprises at least the hexapeptide defined by the sequence SEQ ID No. 2 in which the amino acid of the N-terminal end is acetylated and the amino acid of the C-terminal end is amidated, and at least magnesium gluconate.

8. Composition according to any one of Claims 1 to 7, **characterized in that** the said peptide or peptide mixture represents from 0.000001% to 1% of the total weight of the composition.

9. Composition according to any one of Claims 1 to 8, **characterized in that** the said peptide or peptide mixture represents from 0.00001% to 0.01% of the total weight of the composition.

10. Composition according to any one of Claims 1 to 9, **characterized in that** the calcium-channel inhibitor represents from 0.0001% to 10% of the total weight of the composition.

11. Composition according to any one of Claims 1 to 10, **characterized in that** the calcium-channel inhibitor represents from 0.01% to 1% of the total weight of the composition.

12. Composition according to any one of Claims 1 to 11, **characterized in that** the said composition also contains at least one active agent chosen from: desquamating agents; moisturizers; depigmenting or propigmenting agents; anti-glycation agents; NO-synthase inhibitors; agents for stimulating the synthesis of dermal or epidermal macromolecules and/or for preventing their degradation; agents for stimulating fibroblast and/or keratinocyte proliferation or for stimulating keratinocyte differentiation; dermorelaxants; tensioning agents; antipollution agents and/or free-radical scavengers; agents that act on the capillary circulation; agents that act on the energy metabolism of cells; and mixtures thereof.

13. Cosmetic use (i) of at least one peptide or peptide mixture as defined in one of Claims 1 to 4 and (ii) of at least one calcium-channel inhibitor as defined in one of Claims 1, 5 or 6, in a composition suitable for topical application to the skin, as an agent for preventing or treating, in particular for smoothing out, wrinkles and fine lines.

14. Use according to Claim 13, **characterized in that** the said wrinkles are expression wrinkles.

15. Cosmetic process for treating wrinkled skin, comprising the topical application to the said skin of a composition according to any one of Claims 1 to 12.

16. Process according to Claim 15, **characterized in that** the said composition is applied to the areas of the face or forehead marked with expression wrinkles and fine lines and/or to individuals with expression wrinkles and fine lines.

17. Process according to either of Claims 15 and 16, **characterized in that** the said composition is applied to the wrinkles and fine lines lying radially around the mouth and/or the eyes and/or horizontally on the forehead and/or located in the space between the eyebrows.

## Patentansprüche

1. Zusammensetzung, die für eine topische Anwendung auf die Haut geeignet ist und in einem physiologisch akzeptablen Medium (i) mindestens ein Peptid oder ein Gemisch von Peptiden mit einem Peptid, das unter den in den SEQ ID NO: 2 und SEQ ID NO: 3 definierten Peptiden oder unter
- einem Peptid, das im Wesentlichen homolog zu dem in SEQ ID NO: 2 definierten Peptid ist;
- einem kosmetisch akzeptablen Salz des in der SEQ ID NO: 2 definierten Peptids;
- einem Peptid, das in der SEQ ID NO: 2 definiert ist und reversibel chemisch modifiziert wurde, ausgewählt ist; und (ii) mindestens einen Calciumkanal-Blocker enthält.

2. Zusammensetzung nach Anspruch 1, wobei das Peptid das in der SEQ ID NO: 2 definierte Hexapeptid ist.

3. Zusammensetzung nach Anspruch 1, in der das Gemisch von Peptiden ausgewählt ist unter:
a. einem Gemisch von Peptiden, das mindestens ein Peptid, das unter den Peptiden, die in der SEQ ID NO: 2 und SEQ ID NO: 3 definiert sind, ausgewählt ist, und mindestens ein Peptid mit 3 bis 30 Aminosäuren enthält, das in der SEQ ID NO: 4 enthalten ist;
b. einem Gemisch von Peptiden, das mindestens ein Peptid, das unter den in der SEQ ID NO: 2 und SEQ ID NO: 3 definierten Peptiden ausgewählt ist, und mindestens ein Peptid enthält, das unter den Peptiden ausgewählt ist, die in den SEQ ID NO: 4 und SEQ ID NO: 6 definiert sind.

4. Zusammensetzung nach einem der Ansprüche 1 bis 3, wobei die Aminosäure des N-terminalen Endes des Peptids, wie es in einem der Ansprüche 1 bis 3 definiert ist, acetyliert ist und/oder die Aminosäure seines C-terminalen Endes amidiert ist.

5. Zusammensetzung nach einem der Ansprüche 1 bis 4, **dadurch gekennzeichnet, dass** der Calciumkanal-Blocker unter Alverine und/oder seinen Salzen, Mangan und/oder seinen, Salzen, Magnesium und/oder seinen Salzen ausgewählt ist.

6. Zusammensetzung nach einem der Ansprüche 1 bis 5, **dadurch gekennzeichnet, dass** es sich bei dem Calciumkanal-Blocker um ein Magnesiumsalz handelt, das unter Magnesiumgluconat, Magnesiumsulfat, Magnesiumaspartat, Magnesiumchlorid und Magnesiumpidolat ausgewählt ist.

7. Zusammensetzung nach einem der Ansprüche 1 bis 6, **dadurch gekennzeichnet, dass** sie mindestens ein in der SEQ ID NO: 2 definiertes Hexapeptid, bei dem die Aminosäure des N-terminalen Endes acetyliert und die Aminosäure des C-terminalen Endes amidiert ist, und zumindest das Magnesiumgluconat enthält.

8. Zusammensetzung nach einem der Ansprüche 1 bis 7, **dadurch gekennzeichnet, dass** das Peptid oder das Gemisch von Peptiden 0,000001 bis 1 % des Gesamtgewichts der Zusammensetzung ausmacht.

9. Zusammensetzung nach einem der Ansprüche 1 bis 8, **dadurch gekennzeichnet, dass** das Peptid oder Gemisch von Peptiden 0,00001 bis 0,01 % des Gesamtgewichts der Zusammensetzung ausmacht.

10. Zusammensetzung nach einem der Ansprüche 1 bis 9, **dadurch gekennzeichnet, dass** der Calciumkanal-Blocker 0,0001 bis 10 % des Gesamtgewichts der Zusammensetzung ausmacht.

11. Zusammensetzung nach einem der Ansprüche 1 bis 10, **dadurch gekennzeichnet, dass** der Calciumkanal-Blocker 0,01 bis 1 % des Gesamtgewichts der Zusammensetzung ausmacht.

12. Zusammensetzung nach einem der Ansprüche 1 bis 11, **dadurch gekennzeichnet, dass** die Zusammensetzung ferner mindestens einen Wirkstoff enthält, der ausgewählt ist unter: abschuppenden Wirkstoffen; Hydratisierungsmitteln; depigmentierenden Wirkstoffen oder pigmentierungsfördernden Wirkstoffen; Anti-Glycation-Wirkstoffen; NO-Synthase-Inhibitoren; Wirkstoffen, die die Synthese von Makromolekülen der Dermis oder Epidermis stimulieren und/oder ihre Zersetzung verhindern; Wirkstoffen, die die Proliferation von Fibroblasten und/oder Keratinocyten stimulieren oder die Differenzierung von Keratinocyten stimulieren; dermo-relaxierenden Wirkstoffen, straffenden Wirkstoffen; Antipollutions-Wirkstoffen und/oder Radikalfängern für freie Radikale; Wirkstoffen, die auf die Mikrozirkulation einwirken; Wirkstoffen, die auf den Energiestoffwechsel der Zellen einwirken; und deren Gemischen.

13. Kosmetische Verwendung (i) mindestens eines Peptids oder Gemisches von Peptiden, wie sie in einem der Ansprüche 1 bis 4 definiert sind, und (ii) mindestens eines Calciumkanal-Blockers, wie er in einem der Ansprüche 1, 5 oder 6 definiert ist, in einer Zusammensetzung, die für eine topische Anwendung auf die Haut geeignet ist, als Wirkstoff zur Vorbeugung oder Behandlung und insbesondere Glättung von Falten und Fältchen.

14. Verwendung nach Anspruch 13, **dadurch gekennzeichnet, dass** die Falten Mimikfalten sind.

15. Verfahren zur kosmetischen Behandlung von Haut, die Falten aufweist, das das topische Aufbringen einer Zusammensetzung nach einem der Ansprüche 1 bis 12 auf die Haut umfasst.

16. Verfahren nach Anspruch 15, **dadurch gekennzeichnet, dass** die Zusammensetzung auf die Bereiche des Gesichts oder der Stirn aufgetragen wird, die Mimikfalten und -fältchen aufweisen, und/oder bei Personen, die Mimikfalten und -fältchen haben.

17. Verfahren nach einem der Ansprüche 15 oder 16, **dadurch gekennzeichnet, dass** die Zusammensetzung auf Falten und Fältchen aufgetragen wird, die radial um den Mund und/oder die Augen und/oder horizontal an der Stirn verlaufen und/oder sich im Bereich zwischen den Augenbrauen befinden.


### LISTAGE DES SEQUENCES

<110> L'OREAL
<120> UTILISATION D'UNE ASSOCIATION DE COMPOSANTS A EFFET SYNERGIQUE INHIBITEUR DE CANAUX CALCIQUES POUR PREVENIR OU TRAITER LES RIDES ET LES RIDULES
<130> PR2002.173
<160> 6
<170> PatentIn version 3.1
<210> SEQ ID N°1
   <211> 82
   <212> PRT
   <213> Artificial sequence
   <221> Peptide
<400> 1
   Ala Glu Asp Ala Asp Met Arg Asn Glu Leu Glu Glu Met Gln Arg Arg
   1               5                   10                  15
   Ala Asp Gln Leu Ala Asp Glu Ser Leu Glu Ser Thr Arg Arg Met Leu
               20                  25                  30
   Gln Leu Val Glu Glu Ser Lys Asp Ala Ile Arg Thr Leu Val Met Leu
           35                  40                  45
   Asp Glu Gln Gly Glu Gln Leu Glu Arg Ile Glu Glu Gly Met Asp Gln
       50                  55                  60
   Ile Asn Lys Asp Met Lys Glu Ala Glu Lys Asn Leu Thr Asp Leu Gly
   65                  70                  75                  80
   Lys Phe
<210> SEQ ID N°2
   <211> 6
   <212> PRT
   <213> Artificial sequence
   <221> Peptide
<400> 2
   Glu Glu Met Gln Arg Arg
   1               5
<210> SEQ ID N°3
   <211> 13
   <212> PRT
   <213> Artificial sequence
   <221> Peptide
<400> 3
   Glu Leu Glu Glu Met Gln Arg Arg Ala Asp Gln Leu Ala
   1               5                   10
<210> SEQ ID N°4
   <211> 86
   <212> PRT
   <213> Artificial sequence
   <221> Peptide
<400> 4
   Val Asp Glu Arg Glu Gln Met Ala Ile Ser Gly Gly Phe Ile Arg Arg
   1               5                   10                  15
   Val Thr Asn Ala Arg Glu Asn Glu Glu Met Asp Glu Asn Leu Glu Gln
               20                  25                  30
   Val Ser Gly Ile Leu Gly Asn Leu Arg His Met Ala Leu Asp Met Gly
           35                  40                  45
   Asn Glu Ile Asp Thr Gln Asn Arg Gln Ile Asp Arg Ile Met Glu Lys
       50                  55                  60
   Ala Asp Ser Asn Lys Thr Arg Ile Asp Glu Ala Asn Gln Arg Ala Thr
   65                  70                  75                  80
   Lys Met Leu Gly Ser Gly
                   85
<210> SEQ ID N°5
   <211> 18
   <212> PRT
   <213> Artificial sequence
   <221> Peptide
<400> 5
   Arg Ile Met Glu Lys Ala Asp Ser Asn Lys Thr Arg Ile Asp Glu Ala
   1               5                   10                  15
   Asn Gln
<210> SEQ ID N°6
   <211> 19
   <212> PRT
   <213> Artificial sequence
   <221> Peptide
<400> 6
   Ala Asp Ser Asn Lys Thr Arg Ile Asp Glu Ala Asn Gln Arg Ala Thr
   1               5                   10                  15
   Lys Met Leu